(19)

**Europäisches Patentamt**
**European Patent Office**
**Office européen des brevets**

(11) **EP 3 709 874 B1**

(12) **EUROPEAN PATENT SPECIFICATION**

(45) Date of publication and mention
of the grant of the patent:
**11.06.2025 Bulletin 2025/24**

(21) Application number: **18878926.7**

(22) Date of filing: **17.11.2018**

(51) International Patent Classification (IPC):
**A61B 18/12** (2006.01)    **A61B 18/14** (2006.01)
**A61N 1/05** (2006.01)    **A61N 1/36** (2006.01)

(52) Cooperative Patent Classification (CPC):
**A61B 18/1206; A61B 18/1485;** A61B 18/1402;
A61B 2018/0016; A61B 2018/005;
A61B 2018/00517; A61B 2018/00523;
A61B 2018/00553; A61B 2018/00559;
A61B 2018/00642; A61B 2018/00672;
A61B 2018/00678; A61B 2018/00702;
A61B 2018/00714; A61B 2018/00791;    (Cont.)

(86) International application number:
**PCT/US2018/061706**

(87) International publication number:
**WO 2019/099962 (23.05.2019 Gazette 2019/21)**

(54) **APPARATUS FOR TREATING FEMALE URINARY INCONTINENCE AND FECAL INCONTINENCE**

VORRICHTUNG ZUR BEHANDLUNG VON HARNINKONTINENZ UND STUHLINKONTINENZ BEI FRAUEN

APPAREIL DE TRAITEMENT DE L'INCONTINENCE URINAIRE FÉMININE ET DE L'INCONTINENCE FÉCALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.11.2017   US 201762587766 P**
       **23.04.2018   US 201862661526 P**

(43) Date of publication of application:
**23.09.2020 Bulletin 2020/39**

(73) Proprietors:
• **Thomas, Sherry**
  **Agoura Hills, CA 91031 (US)**
• **Koop, Dale**
  **Tracy, CA 95376 (US)**

(72) Inventors:
• **Thomas, Sherry**
  **Agoura Hills, CA 91031 (US)**

• **Koop, Dale**
  **Tracy, CA 95376 (US)**

(74) Representative: **Wall, Leythem**
  Oxon IP B.V.
  **Evert Van De Beekstraat 354**
  **1118 CZ Schiphol (NL)**

(56) References cited:
EP-A1- 3 195 898       WO-A1-00/71043
WO-A1-2017/066625   WO-A2-2007/092610
WO-A2-2011/034986   US-A1- 2011 178 584
US-A1- 2012 323 227   US-A1- 2013 245 728
US-A1- 2016 121 112   US-A1- 2016 184 015
US-A1- 2016 263 388   US-A1- 2017 209 707
US-B1- 6 470 219       US-B2- 7 699 844
US-B2- 9 713 565

**(Cont. next page)**

(52) Cooperative Patent Classification (CPC): (Cont.)
A61B 2018/00875; A61B 2018/1467; A61N 1/0512;
A61N 1/0524; A61N 1/36007

# EP 3 709 874 B1

## Description

BACKGROUND OF THE INVENTION

### 1. FIELD OF THE INVENTION:

This invention relates to the field of medical treatments. More particularly, this invention relates to the field of treating urinary and fecal incontinence, pelvic prolapse and pelvic pain and other medical conditions, and a device for treating those conditions.

### 2. DESCRIPTION OF RELATED ART

A number of different methods have been proposed to treat female urinary incontinence. Non-surgical methods that have been proposed include electrical stimulation, behavioral modification, pelvic floor strengthening via exercises and vaginal weight cones, and medication (estrogen and a-adrenergic agonists). Surgical methods include surgical implants with slings materials using polypropylene mesh, periurethral injection with various materials including Polytef paste and GAX collagen. Other injectables include autologous fat tissue and silicone microimplants. Injectable agents have been used to manage stress urinary incontinence for more than 50 years, but have limited application due to infection, placement limitations, long term durability, antigenicity, and other compatibility issues.

For fecal incontinence, various treatments have been proposed including biofeedback, sphincteroplasty, anal dynamic graciloplasty, and external anal sphincter plication with or without pelvic floor repair.

US2016/263388 A1 and WO2007/092610 A2 disclose prior art devices for treating female urinary incontinence and fecal incontinence.

### SUMMARY OF THE INVENTION

The present invention is of a medical device of treating urinary incontinence, fecal incontinence, and other medical conditions.

The invention is defined in appended independent claim 1, further embodiments are described in the dependent claims.

The surgical methods described below are not claimed and are not part of the present invention.

An exemplary method of treating urinary incontinence involves using radiofrequency (RF) energy to heat target tissue to a temperature that is sufficient to simulate fibroblasts within the body to produce mostly type 1 collagen without burning the tissue or stimulating the production of mostly type 3 collagen. The target tissue includes: tissue surrounding the urethra, tissue within the urethra, and the perineum.

An exemplary method of treating fecal incontinence involves using RF energy to heat target tissue to a temperature that is sufficient to simulate fibroblasts within the body to produce mostly type 1 collagen without burning the tissue or stimulating the production of mostly type 3 collage. The target tissue includes: the perineum, tissue surrounding the rectum, the internal rectal sphincter, the external rectal sphincter, and in women the inside vaginal area.

An exemplary device for applying the RF energy includes a grid of monopolar microelectrodes that is sometimes called a grid fractional applicator, or a fractional RF array. The grid fractional applicator spreads out the RF energy to protect the epidermis and creates a uniform thermal zone in the dermis for safe, reproducible and consistent results. Because the RF energy is divided among many micro electrodes, there is no risk of burns due to RF arcing between the electrode and skin.

An exemplary method of treating fecal incontinence in a patent, the patient having an anus, the anus including anal tissue, comprises: raising the temperature of said anal tissue to greater than 40° C. Said anal tissue may be raised to a temperature of between 40° C and a maximum of 45° C. Said anal tissue may comprise internal anal sphincter tissue. Said anal tissue may comprise external anal sphincter tissue. Said anal tissue may comprise tissue within a perineum of the patent, with the method further comprising heating tissue within the perineum to a temperature of greater than 40° C. Said raising of the temperature may comprise heating using a fraction RF array to delivery radiofrequency energy to the anal tissue. Said fractional RF array may have at least 9 electrodes distributed over an area of at least 1 cm$^2$.

An exemplary medical device for at least one of treating female urinary incontinence, treating fecal incontinence, and vaginoplasty comprises: an elongated wand portion having a generally cylindrical shape, the wand having a proximal end and a distal end; a fractional radiofrequency (RF) array proximate the distal end of the wand, the fractional RF array having a plurality of electrodes distributed over an area of greater than 1 cm$^2$ for delivering RF energy to a target tissue of a patient; and dielectric material covering one or more electrodes within the RF array such that said one or more electrodes are electrically insulated from the target tissue of the patient, and energy is transmitted from the one or more electrodes to said target tissue of the patient via capacitive coupling. Said fractional RF array may have at least 9 electrodes distributed over an area of at least 1 cm$^2$. Said medical device may further comprise: an impedance sensor for sensing an impedance from the fractional RF array through the patient, the impedance as sensed constituting a sensed impedance; and a control circuit operatively connected to the fractional RF array for adjusting an amount of RF energy provided to the fractional RF array based on the sensed impedance such that the target tissue is kept to a temperature below 48° C. Said medical device may further comprise: a plurality of visible circumferential gradations on said elongated wand portion, the circumferential gradations being spaced a distance apart that approximately equals a width of said fractional RF array, such that when the fractional RF array is inserted inside a patient a doctor can rotate the wand by one circumferential gradation at a time for achieving substantial uniform RF energy exposure of the target tissue as the wand is rotated without the doctor being able to see either the fractional RF array or the target tissue that is being exposed to the RF energy. Said visible circumferential gradations may comprise grooves. Said medical device may further comprise: a plurality of visible longitudinal gradations on said elongated wand portion, the longitudinal gradations being spaced a distance apart that approximately equals a longitudinal length of said fractional RF array, such that when the fractional RF array is inserted inside a patient a doctor can insert or retract the wand one longitudinal gradation at a time for achieving substantial uniform RF energy exposure of the target tissue without being able to see either the fractional RF array or the target tissue that is being exposed to the RF energy. Said fractional RF array may be flat. Said fractional RF array may be curved. Said electrodes may comprise monopolar electrodes. Said electrodes may comprise bipolar electrodes. Said medical device may further comprise: an impedance sensor for measuring an impedance from said electrodes through said target tissue; and a feedback control loop for controlling how much RF energy is delivered through said electrodes in order to maintain a temperature of said target tissue at a desired temperature. Said feedback control loop may maintain a temperature of said target tissue of 40° to 45° C.

Exemplary embodiments of the invention will be further described below with reference to the drawings, in which like numbers refer to like parts. The drawing figures might not be to scale, and certain components may be shown in generalized or schematic form and identified by commercial designations in the interest of clarity and conciseness.

BRIEF DESCRIPTION OF THE DRAWINGS

FIG. 1 is a perspective view of an illustrative embodiment of the medical device of the present invention.

FIG. 2 is a closeup of the electrode of the medical device of **FIG. 1.**

FIG. 3 is a closeup of the proximal end of the medical device of **FIG. 1** showing the electrical contacts thereof.

FIG. 4 is a side view of the electrode of the medical device of **FIG. 1.**

FIG. 5 is a simplified diagram of the pelvic region of a female patient, illustrating exterior areas that are suitable for treatment according to the present invention.

FIG. 6 is a graph of patient data for vaginal laxity treatment according to the present invention.

FIG. 7 is a graph of patient data for urologic distress treatment according to the present invention.

FIG. 8 is a graph of patient data for urologic distress treatment according to the present invention.

FIG. 9 is a graph of patient data for sexual dysfunction treatment according to the present invention.

FIG. 10 is a graph of patient data for sexual desire treatment according to the present invention.

FIG. 11 is a graph of patient data for sexual arousal treatment according to the present invention.

FIG. 12 is a graph of patient data for sexual satisfaction treatment according to the present invention.

FIG. 13 is another graph of patient data for sexual satisfaction treatment according to the present invention.

DETAILED DESCRIPTION OF THE PREFERRED EMBODIMENTS

1. The Device

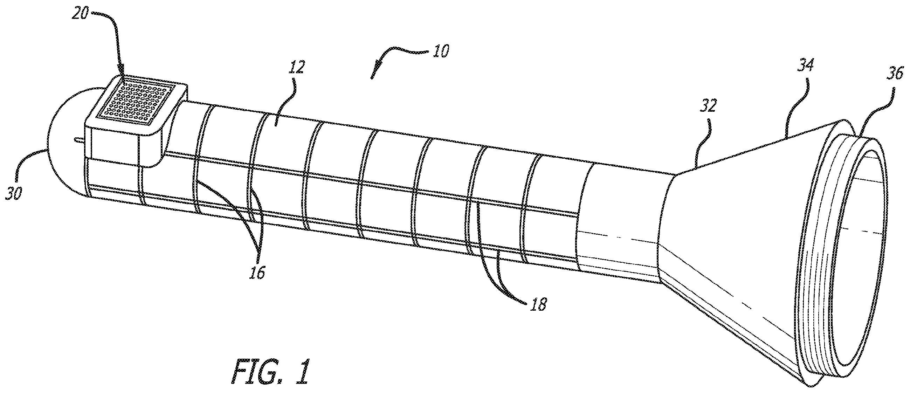

**FIG. 1** is a perspective view of an illustrative embodiment of the medical device 10 of the present invention. The device 10 includes a wand portion 12 having a smoothly rounded proximal end 30 and an electrode (e.g., a fractional RF array) 20, which may be a grid or plate, and a distal end 30. At a proximal end 32 of the wand portion 12 is a generally conical section 34 having a thread 36 so that the electrical cable which supplies the input electrical signal to the electrode 20 can be securely fastened to wand portion 12. For definitional purposes, the proximal end 32 is the end at which the doctor or other treating professional holds the device, and the distal end 30 is the end that is opposite the distal end 32. The wand portion 12 is approximately 14.5 cm long and approximately 2.5 cm wide in one embodiment. More generally, the wand portion can be approximately 8 - 20 cm long and approximately 1.5 - 4 cm wide.

The wand portion 12 has a number of visible longitudinal gradations 16 marked on it, as well as a number of visible circumferential gradations 18 marked on it, whose use will be explained later. The circumferential gradations 18 are spaced at regular intervals that approximately equal the transverse width of the electrode 20. In the illustrative embodiment there are 5 circumferential gradations 18 spaced equally around the circumference of the device (which can be marked 0 thru 4 or in some other suitable manner).

The longitudinal gradations 16 also can be marked at regular intervals. The longitudinal gradations are spaced at regular intervals that approximately equal the longitudinal length of the electrode 20. In the illustrative embodiment the gradations 16, 18 are grooves in the surface of wand portion 12, but could be printed or otherwise marked.

As mentioned above, at the proximal end 32 of the device 10, the conical portion 34 has a thread 36 near the far end. The thread 36 allows an electrical connection cable (not shown in **FIG. 1**) to be securely attached via a freely rotating threaded ring on the cable that mates with the thread 36 thereby securing the cable to the device 10. Such mating threads to secure electrical connections are well known.

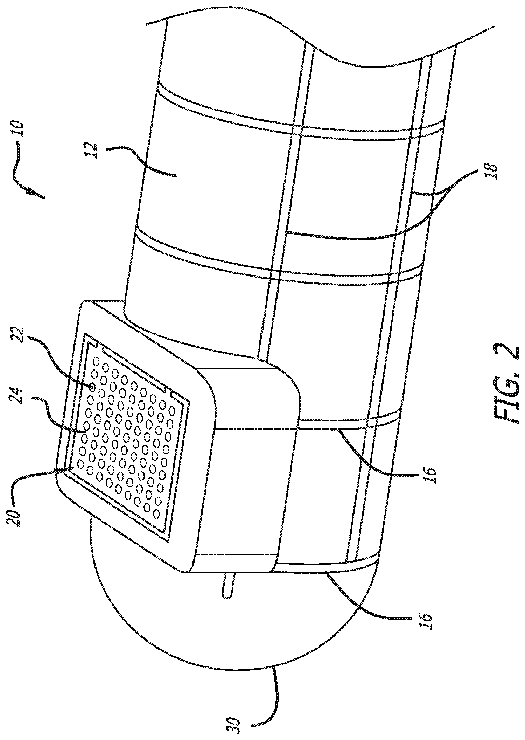

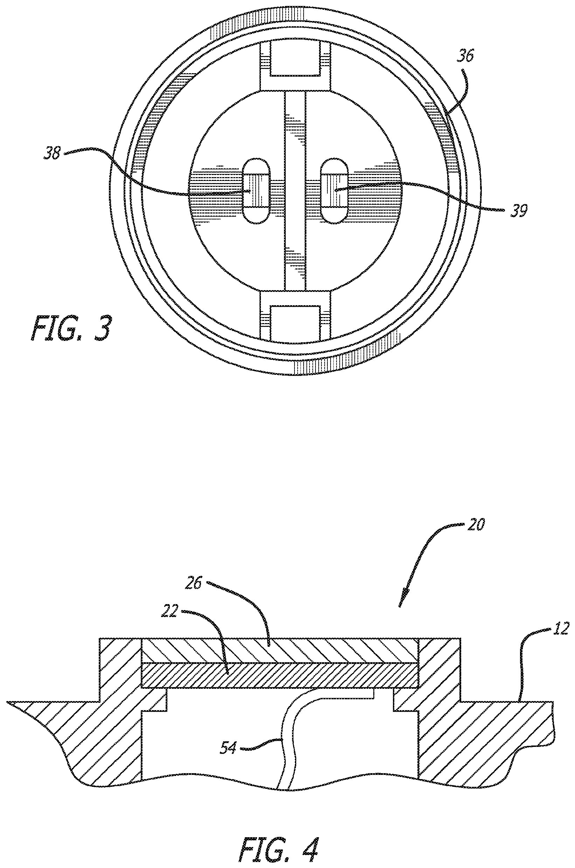

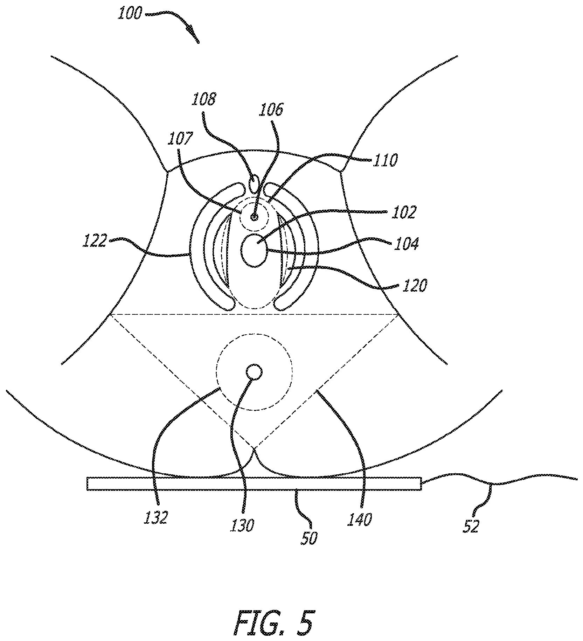

**FIG. 2** is a closeup of the electrode 20 of the medical device 10 of **FIG. 1**. The electrode 20 may take the form of a screen with commonly connected electrode elements 22 with spaces 24 between the electrode elements 22. In use, the electrode elements 22 within the electrode 20 do not directly touch the patient's skin or other target tissue. That is, the electrode elements 22 are not placed in direct electrical contact with the skin. Rather, the electrode elements 22 are covered by an electrical insulator 26 (see **FIG. 4**), preferably a dielectric and non-toxic, high temperature resistant, medical grade material such as polyimide (e.g., KAPTON®), such that direct electrical current does not pass from the electrodes to the target tissue. The electrode 20 emits electromagnetic radiation, preferably in the RF spectrum such as approximately 1 MHZ, 2 MHz , or 6 MHz, which electromagnetic radiation causes heating within the target tissue. Additionally, alternating current (AC) is capacitively coupled between the electrode elements 22 within the electrode 20 and the target tissue, also heating the target tissue. Because the electrodes are monopolar, the device is used with a metal ground plate 50 (see **FIG. 5**) that is applied to the patient such as on the patient's thigh or buttock during treatment and in direct electrical contact therewith. Ground potential is provided to the ground plate 50 via a ground lead wire 52 (see **FIG. 5**).

Because the energy that is applied to the electrode 20 is capacitively coupled into the target tissue rather than electrical current flowing from electrode 20 to the tissue, the electrode elements 22 together act as a capacitor charge plate, with the electrical charge spreading out uniformly over the entire plate, and the energy being transmitted to the target tissue via electromagnetic emission in the RF spectrum. This creates a much more uniform application of RF energy to the target tissue over the entire area of electrode 20. In contrast, if electrical current were to flow from the electrode 20 to the target tissue, the current would have a tendency to flow through the path of least resistance, with the result being that the current flow would occur more or less at a single point. Because the energy is spread out and transmitted uniformly, the doctor can hold the electrode 20 over a single spot on the tissue, apply one or more metered pulses of energy, and know exactly how much energy has been applied to the target tissue and know that the energy was applied uniformly over the area of the electrode 20.

This allows a system that includes the device 10 and a controller to monitor the tissue that is being treated by using impedance sensing/feedback, and adjust the amount of energy being delivered during the actual treatment.

In contrast, other systems that use impedance monitoring are actually monitoring the impedance of tissue that has already been treated. Such systems can monitor an average impedance within the tissue, but they cannot compensate and adjust the energy for the tissue that is about to be treated.

A system based on the present invention can monitor impedance and adjust the power level several times during a pulse. For example, such a system can monitor the impedance, adjust the energy, deliver the energy, then monitor the impedance again, and adjust the energy. The system can therefore fine-tune the amount of energy delivered into tissue so that all tissue in the treated area receives the same amount of energy and receives the same amount of heat. Thus, the system effectively acts like a dermal thermostat. As energy is delivered to tissue, the tissue's characteristics change. The resistance of human tissue decreases with increasing temperature.

For rapidly moving electrodes as used in some prior art treatment methods, it is difficult to adjust the amount of energy delivered based on temperature of the target tissue. Such systems commonly employ constant voltage or constant current and as the electrodes move over tissue, tissue that has been heated before will have less resistance and hence will absorb more energy than tissue that has not been treated, increasingly channeling the electrical current through the spots that have already been treated and away from the areas that have not been treated. The tissue will develop hotter and hotter hot spots and colder and colder cold spots, but on the average the temperature sensor will measure a uniform temperature because the doctor or other treating professional is moving the probe rapidly over tissue and the temperature monitors are averaging out the inconsistencies.

In contrast, by employing a feedback control loop, a system which uses the electrode 20 can adjust the energy for the optimum amount to be delivered to the target tissue, resulting in a more comfortable and uniform treatment.

In the illustrative embodiment, the device 10 uses monopolar electrodes together with a ground plate 50 as discussed herein. Alternatively, the device could use bipolar, tripolar, qudrapolar, tetrapolar, or any combination of electrodes including fractional bipolar electrodes.

Preferably the device includes a screen electrode or grid electrodes (e.g., 9 spaced commonly connected electrodes 22) distributed over an area of at least 1 cm$^2$, and more preferably a screen electrode of grid electrodes (e.g., 25 spaced commonly connected electrodes 22) distributed over an area of at least 2 cm$^2$.

In the exemplary embodiment, the electrode 20 is a grid of monopolar microelectrodes that is sometimes called a grid fractional applicator. Such an electrode is currently manufactured by Jesisys Medical, Inc. of Korea ("Jesisy Medical") and designated by model number KT-15 for a 15 mm x 15 mm grid array. The KT-15 grid array uses an array of 9 x 9 commonly connected electrodes potted in a polymer for a total of 81 fractional electrodes over an area of approximately 2.25 cm. As used herein, the grid acts as a plate except at the immediate surface where the field concentrated at the grid wires leaves a fractional grid thermal pattern in the epidermis to cause fractional resurfacing of the epidermis.

**FIG. 3** is a closeup of the proximal end 32 of the medical device of **FIG. 1** showing electrical contacts 38 and 39 thereof. An electrical cable leading to a control unit (not shown in **FIG. 1**) including a pulse generator physically and electrically mates with this end of the device.

**FIG. 4** is a side view of the electrode 20 of the medical device 10 of **FIG. 1.** As shown, the electrode 20 may include electrode elements 22 (e.g., a metal screen electrode which acts as a plate electrode) covered by the electrical insulator 26. In addition, a conductor 54 provides an electrical connection to a signal generator (not shown in FIG. 4).

The electrode 20 and device 10 of the present invention differ markedly from prior RF treatment devices such as the one described in U.S. Patent No. 8,540,705 to Mehta and sold by Syneron Medical Ltd. aka Syneron Candela, in that those prior devices feature an array of microneedles that penetrate the skin and thus deliver RF energy subcutaneously, delivering the energy through the sharp end point of the needle, and to a ground pad applied to the patient. The electrical current of those devices flow through the well-defined tip of the needle into tissue, flowing primarily through the path of least electrical resistance from the tip of the needle to a sharp edge or protrusion of corner of the ground pad, providing imprecise electrocautery.

The electrode 20 and device 10 also provide a more uniform treatment field than prior devices such as disclosed in U.S. Patent No. 8,961,511 to Palmer and corresponding devices sold by Viveve, Inc. In those devices electrical current flows from the electrodes through the target tissue and to a ground plate. Again, the electrical current will choose the path of least resistance, such that the electrocautery is more localized than desired. Thus, the doctor must move those devices around rapidly over the target tissue to prevent burning. When moving a wand around rapidly over a surface to treat the surface uniformly the doctor is unable to determine fluence at any point in the tissue, and it can be difficult for the doctor to obtain consistent results.

The exemplary device 10 also has an electrical impedance sensor or monitor for indirectly sensing the temperature of the tissue to which the device is applied, by measuring tissue impedance and using known relationships between tissue impedance and tissue temperature. The device 10 includes a control circuit operatively connected to the impedance monitor and the electrode 20 to prevent the tissue from being heated beyond a predetermined maximum temperature. The impedance monitor indirectly senses the tissue temperature and provides feedback to a power control circuit. The relationship between skin temperature and electrical impedance is well known. See for example, R. Gudivaka et al, "Effect of Skin Temperature on Multifrequency Bioelectrical Impedance Analysis," Journal of Applied Physiology, August 1996, 81(2): 838-45. Current through the electrode 20 and into the human body quickly spreads out in the body providing a wide electrical path, such that the impedance of the heated tissue immediately adjacent the electrode 20 constitutes the major contributor to the total impedance sensed, and thus the temperature of that heated tissue can be accurately calculated.

The electrode 20 (e.g., the model KT-15 RF grid array) indirectly measures temperature of the target tissue by measuring electrical impedance through the body including through the target heated tissue to which it is applied. The impedance monitor provides feedback to the control circuit which can actively adjust the RF power applied to maintain a controlled energy dose for a constant temperature rise. A control circuit for monitoring the impedance and for adjusting the power applied to the electrode 20 to achieve the desired temperature of the target tissue may be located in an external unit that generates the electrical input to the device 10 and hence the electrode 20. The control circuit may apply one or multiple pulses of energy at spaced intervals such as regular intervals to first bring the target tissue to the target temperature and then to hold it at the target temperature for the desired time. The control circuit may therefore apply energy at a higher average power for a short time duration initially to quickly bring the target tissue to the desired temperature and then reduce the average power to maintain the target tissue at that temperature for the desired time. The control circuit may adjust any or more of the following parameters to achieve the desired treatment profile: instantaneous power, "On" time, and duty cycle.

The device could use means other than RF energy for raising the temperature of the target tissue including but not limited to direct heating, and electromagnetic waves within the microwave spectrum or other frequency spectrum.

It is preferred that the temperature of the target tissue during treatment be within the range of 40° C - 48° C, and more preferably within the range of between 40° C and a maximum of 45° C, and more preferably still to within the range of 43° C - 44° C. Above 45° C the cells begin to burn, resulting in production of too much type 3 collagen which is scar tissue. Keeping the temperature of the target temperature to within the range of about 40° C to 45° C stimulates the fibroblasts and thus stimulates the production of mostly type 1 collagen which is has better elasticity than type 3 collagen, and results in only partial denaturation of the existing collagen. RF energy produces heat which promotes neocollagenesis, denaturation of collagen cross-links, activation of wound healing pathways, contraction of collagen, and increase in collagen fibril size. The phenomenon of thermal contraction of collagen begins with a denaturation of the triple helix of the collagen molecule. Partial denaturation of collagen tissue results in a contraction of the collagen-rich spaces and provides a "tightening" effect on the overlaying tissue.

Heat is the result of RF waves experiencing impedance as the RF waves traverse the tissue bed. As the RF waves permeate a tissue layer, electric fields are induced across the tissue, and ions found within that layer deliver or carry an electrical current. This, in turn, increases the kinetic activity of the ions. Increased ion kinetics and oscillations engenders resistive heating in tissue called thermogenesis heating. The degree of heating is calculable via the Specific Absorption Rate (SAR) equation, which assesses local electrical conductivity and magnitude of local electric current density generated around the electrode:

$$SAR = \frac{\sigma}{\rho}|E|^2 = \frac{1}{\sigma^2 \rho}|J|^2$$

EQUATION 1

The therapeutic benefit is localized thermogenesis heating. The electric field strength generated is capable of heating tissue in close proximity to the electrode. Dispersion of the electric field causes decreasing energy deposition away from the surface of the electrode. With proper controls, generating the ideal thermal endpoint will occur close to the electrode, affecting only a specific tissue.

Grid fractional radio frequency energy creates thermal grid shapes on the surface and a uniform thermal zone in tissue during RF emission. Regulated RF current is adjusted via power control to match the desired impedance, which corresponds via known relationships to tissue temperature. Power absorbed equals the square of the electrical current delivered to by the radiofrequency electrode times the tissue impedance. The tissue impedance will be monitored, and the power will be adjusted to ensure that the proper current is delivered to achieve the desired tissue impedance. In summary, feedback from the impedance sensor is monitored by the control system, and power is adjusted to maintain the therapeutic energy deposition and resultant temperature rise in tissue. Altogether, with these proper controls in place, selective thermogenesis heating serves as a viable treatment for numerous medical conditions.

The variables involved in changing collagen relate to temperature delivered to a specific area of tissue over a certain time. The maximal shrinkage of collagen tightening before the tissue becomes too weak is 15-20% (see Wall, MS, et al. , J Shoulder Elbow Surg.; 1999 Jul-Aug; 8(4):339-44). The shrinkage of collagen is related to time and the amount of energy applied.

Preferably the target tissue is heated to the preferred temperature of 43° C - 44° C for a specified exposure time. This can be accomplished by applying various waveform profiles to the electrode 20.

**FIG. 5** is a simplified diagram of the pelvic region of a female patient 100, illustrating exterior areas that are suitable for treatment according to the present invention.

For treating female urinary incontinence, the target tissue includes: vaginal epithelium in the tissue 107 surrounding the urethral opening 106 but not including tissue within the hymenal ring 104; labial epithelium including of the labia minora 120 and labia majora 122 surrounding the urethral opening 106, and anywhere outside the pelvic floor; and epithelium in the perineum 140. The volume of blood flow within this area is sufficiently high that the electrode 20 can be applied to one area to effectively heat local surrounding tissue that receives the flow of heated blood. Collagen production can thus be stimulated in a surrounding adjacent area other than the area that is immediately underneath or adjacent to the electrode 20.

Also visible in the figure are the vagina 102, clitoris 108, Hart line 110, anus 130, and anal tissue 132.

The marked gradations 16, 18 on the device as seen in **FIG. 1** assist the treating professional in achieving full coverage of the target area without overlap, when the area being treated is within the vagina or within the anus. The longitudinal gradations 16 marked on the device can be used to ensure that the electrode 20 is placed at the desired depths, and the circumferentially marked gradations 18 allow the treating professional to achieve the treatment desired circumferentially (e.g., within the range of 0° - 360° of arc, or only a narrower arc, such as 30° - 330° of arc) to avoid the clitoral area, at a specified depth, obtaining full coverage of the target tissue and without overlap. For example, a treating professional might insert the device up to the first marked longitudinal mark 16, apply energy to the device for a first time period to treat the area of tissue that is touching the electrode 20, rotate the device by one circumferential gradation 18, then apply energy to the device for the same time period, and so on until energy has been applied at that depth around the entire 0° - 360° of arc. Then the treating professional inserts the device to the next longitudinal mark 16, and repeats the treatment within the circumferential range of 0° - 360°. In this way the treating professional applies the electrode 20 to each portion of the treatment area sequentially and achieves full coverage of the target tissue but without overlap, which could cause overexposure and therefore less than ideal response within the target tissue or even burning. In this way the doctor can assure uniform treatment of the entire target area within the patient's vagina, and to a predetermined depth, without having to see the electrode 20 or the tissue to which it is applied. The markings therefore eliminate the guesswork that was necessary with some prior art devices for treating inside body cavities, in which the devices were intended to be swirled rapidly within the body cavity to ensure uniform treatment, and in which it was difficult for the doctor to achieve uniform treatment of the target tissue.

2. Treating Urinary Incontinence

The device 10 may be applied to treat female urinary incontinence by tightening vaginal epithelium around the urethral opening 106, particularly urinary stress incontinence for which common contributing causes include a weakened urethral sphincter and weakened pelvic floor muscles. The etiology of urinary incontinence is multifactorial. Stretching, tearing and separating of the pelvic floor support tissue during childbirth along with other traumatic conditions of chronic coughing, weight bearing and collagen vascular disorders increase the risk of incontinence and pelvic floor prolapse. Other causes include The Genitourinary Syndrome of Menopause (GSM) which is a progressive devastating condition from the reduction of estrogen and blood flow on the urogenital tissue (Portman, 2014, Maturutas). The clinical findings of pale pink vaginal epithelium with flattened dry rugae and associated urinary symptoms of urgency, frequency, incontinence, dysuria, dryness, burning, pain, sexual dysfunction affect up to 50% of menopausal women.

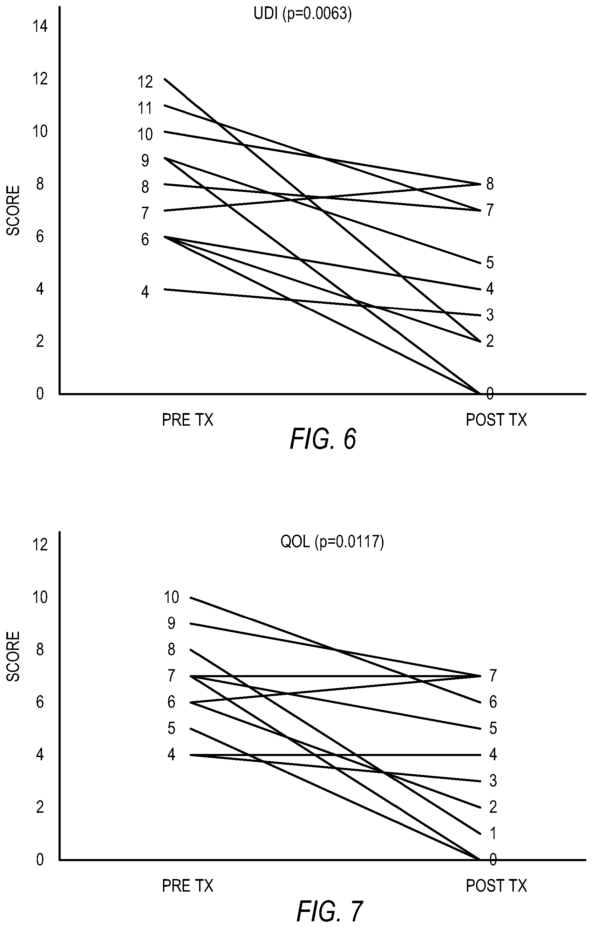

FIGs. 6 and 7 illustrates preliminary data for 14 patients from a clinical trial (NTC03280446 Clinicaltrials.gov). The scores prior to treatment (PRE TX) and after treatment (POST TX) reveal a statically significant improvement on the Urological Distress Inventory (UDI) (FIG. 6), the Incontinence Impact Questionaire (IIQ), and the Urinary Quality of Life Questionaire (QOL) (FIG. 7). In the figures, the parameter "p" relates to the statistical significance (e.g., with $p < 0.05$ indicating statistical significance). The Incontinence QOL Questionaire states: "If you were to spend the rest of your like with your urinary condition just the way it is now, how would you feel about that? Circle the number that best reflects your feelings about your urinary problem. Scores range from 0 to 10, indicating pleased (low scores e.g., 0), indifferent (medium score, e.g., 5), or terrible (high score, e.g., 10).

The treatment device 10 of FIGS. 1-3 can be used to treat the areas discussed above. Additionally, a different and much smaller treatment wand could be used to treat tissue inside the urethra itself. Applying the treatment inside the urethra stimulates the production of collagen there and hence strengthens and tightens the urethral opening.

Although it is believed that the method and device of the present invention will be most effective for treating urinary stress incontinence, it is believe that the method and device will also have efficacy in treating urinary urge incontinence.

The application of radiofrequency to tissue surrounding the urethra and rectal sphincter tightens the supporting tissue by creating new extracellular collagen and elastin matrix. The resulting tighter and better-recoiling tissue will increase the tone and pressure, and coaptic ability within the urethra and rectal sphincter. As a result the patient will experience less incontinence both at rest and with contraction of the pelvic floor.

Some techniques for tissue tightening that have been proposed by others use high temperatures such as up to 85° C and rely heavily on denaturation of existing collagen within the tissue. (see Wall mentioned above). Although denaturation of the collagen triple helix does result in shrinkage of the tissue, it also produces the undesirable effect of reducing the strength of the tissue. Additionally, temperatures above 85° C burn the cells and result in the production of too much type 3 collagen which is scar tissue, and which has little elasticity. The method of the present invention can therefore produce superior results in the tissue targeted as compared to prior results that induced higher target tissue temperatures.

Additionally, prior devices that have been used for various applications employ relatively low power levels of approximately 20 watts. Such low power levels were necessary because electrical current tended to flow out from one localized portion of a much larger metal electrode that was in direct contact with the skin, thus producing heating over a very small area that approached a point heat source. A power of greater than 20 watts presented a threat of burning tissue.

In contrast, the device of the present invention assures that energy will be applied generally uniformly over a relatively large area, namely, over the entire area of the electrode 20. The device is capable of delivering a total power of about 200 watts without risking tissue burning. Even though the target tissue will be brought to a relatively modest temperature of about 40 - 45° C, the high wattage of the present device provides the advantage of providing more uniform energy distribution over a known area of application and using a shorter pulse of RF energy. Because the present invention allows a higher power level, distributing that power over a broader area, the present invention allows full treatment over a given area in less total treatment time that some of the previous devices required.

### 3. Treating Fecal Incontinence

The device 10 including the electrode 20 can also be used for treating fecal incontinence in both male and female patients. For treating fecal incontinence, the same biological thermal responses discussed above apply to this treatment. The target tissue includes: the perineum 140 including perineum epithelium, tissue surrounding the rectum, the internal rectal sphincter, and the external rectal sphincter.

For treating areas in and around the anus 130, it is important to avoid the prostate area in men and the rectal mucosa above the dentate line.

### 4. Vaginal Treatment

The method and device of the present invention could also be used to treat vaginal epithelium within the vagina 102, including vaginal epithelium within the region from about 3.5 cm past the introitus which the area at the vaginal opening to the end of the vagina which is generally about 8-12 cm past the introitus, to achieve vaginoplasty.

The method and device can also be used to treat pelvic pain, including dyspareunia which his painful intercourse.

Chronic pelvic pain is multifactorial and may be caused by several inflammatory processes including chronic infectious cystitis, interstitial cystitis endometriosis and pelvic inflammatory disease. Neurological trauma or diseases in the pelvic floor affecting the innervation of the pudendal, ilioinguinal, iliohypogastric and genitofemoral nerves may also cause pelvic pain conditions from neuropathy and muscle spasms. Pelvic neuralgias are described as causing pelvic pain conditions and associated bladder, bowel, and sexual dysfunctions through direct damage and production of inflammatory processes. The inflammatory mediators may including cytokines, chemokines, free radicals, prostaglandins, cyclooxygenase and matrix metalloproteinase growth and transcription factors, microRNAs, creating a unfriendly microenvironment. Treatment of pelvic pain with physical therapy, nerve blocks has been well described in the literature. Clinical effectiveness with pulsed high-frequency radiofrequency (PRF) treatment applied to the pudendal nerve under ultrasound guidance has been described by Ozkan D, et al., Anaesthesist; 2016 Feb; 65(2):134-6. doi: 10.1007/s00101-015-0133-4; Epub 2016 Jan 26. Radiofrequency.

A treatment method using the device 10 may improve blood flow, reduce muscle spasm and reduce chronic inflammatory diseases by reducing or modulating inflammatory signaling pathways.

The method and device disclosed here may also increase vaginal lubrication. Heating an area temporarily creates improved blood flow temporarily, which promotes angiogenesis which is the growth of new blood vessels. The new blood vessels lead to improved vaginal lubrication. The histology changes on biopsy immediately induce fibroblast stimulation without disruption of the tissue architecture or cell death. The histology 90 days after treatment produces remodeling of tissue with increased type 1 collagen (i.e., cross-linking, increased fiber size) and reducing the quality of older collagen.

### 5. Treating Pelvic Prolapse

The method and device of the present invention could also be used to treat pelvis prolapse. The histology changes in collagen from 3 ovines after radiofrequency treatment to the vaginal introitus is described by Vos et al. The histology changes on biopsy immediately induce fibroblast stimulation without disruption of the tissue architecture or cell death. The histology 90 days after treatment produces remodeling of tissue with increased type 1 collagen (i.e., cross-linking, increased fiber size) and reducing the quality of older collagen. These changes of improved quality and strength of collagen along with the shrinkage of collagen bonds described by Wall (mentioned above) may produce the improvement seen in pelvic prolapse.

After treatment using the method and device of the present invention, patients were examined in the dorsal lithotomy position using the POPQ Staging Criteria measurement system. The patients were again examined at 90 days post treatment. The preliminary data in the initial 22 patients from NTC03280446 Clinicaltrials.gov result reveals a statically significant improvement in the clinical diagnosis of pelvic prolapse.

### 6. Treating Vaginal Laxity

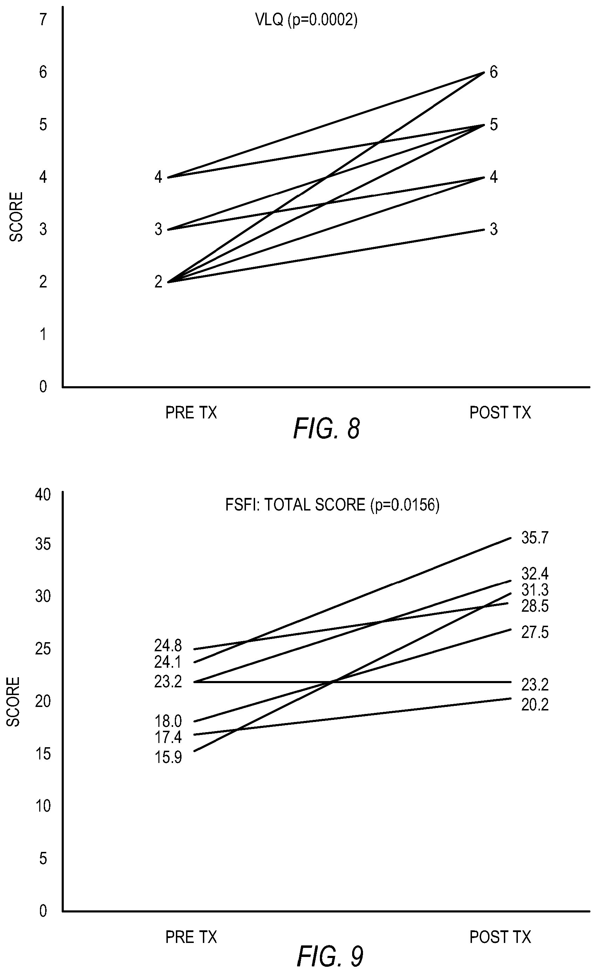

The method and device of the present invention could also be used to treat vaginal laxity (e.g., patients self-reported vaginal laxity). **FIG. 8** illustrates preliminary data for 14 patients from a clinical trial (NTC03280446 Clinicaltrials.gov) using techniques as described herein. The scores prior to treatment (PRE TX) and after treatment (POST TX) reveal a statically significant improvement on the Vaginal Laxity Questionaire (VLQ).

### 7. Treating Sexual Dysfunction

The method and device of the present invention could also be used to treat sexual dysfunction. **FIG. 9** illustrates preliminary data for 14 patients from a clinical trial (NTC03280446 Clinicaltrials.gov) using techniques as described herein. The scores prior to treatment (PRE TX) and after treatment (POST TX) reveal a statically significant improvement on the Female Sexual Function Inventory (FSFI) in 7 of the 14 patients who were diagnosed with sexual dysfunction (FSFI<26.5).

### 8. Treating Female Sexual Desire

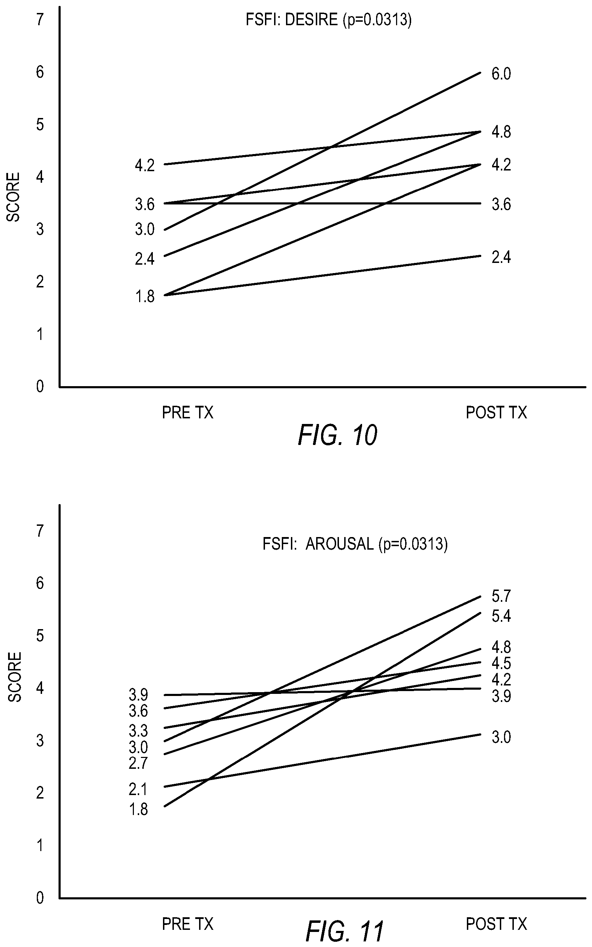

The method and device of the present invention could also be used to treat sexual desire. FIG. 10 illustrates preliminary data for 14 patients from a clinical trial (NTC03280446 Clinicaltrials.gov) using techniques as described herein. The scores prior to treatment (PRE TX) and after treatment (POST TX) reveal a statically significant improvement on the Desire Domain of the Female Sexual Function Inventory (FSFI: Desire).

### 9. Treating Female Sexual Arousal

The method and device of the present invention could also be used to treat sexual arousal. **FIG. 11** illustrates preliminary data for 14 patients from a clinical trial (NTC03280446 Clinicaltrials.gov) using techniques as described herein. The scores prior to treatment (PRE TX) and after treatment (POST TX) reveal a statically significant improvement on the Arousal Domain of the Female Sexual Function Inventory (FSFI: Arousal).

### 10. Treating Female Sexual Satisfaction

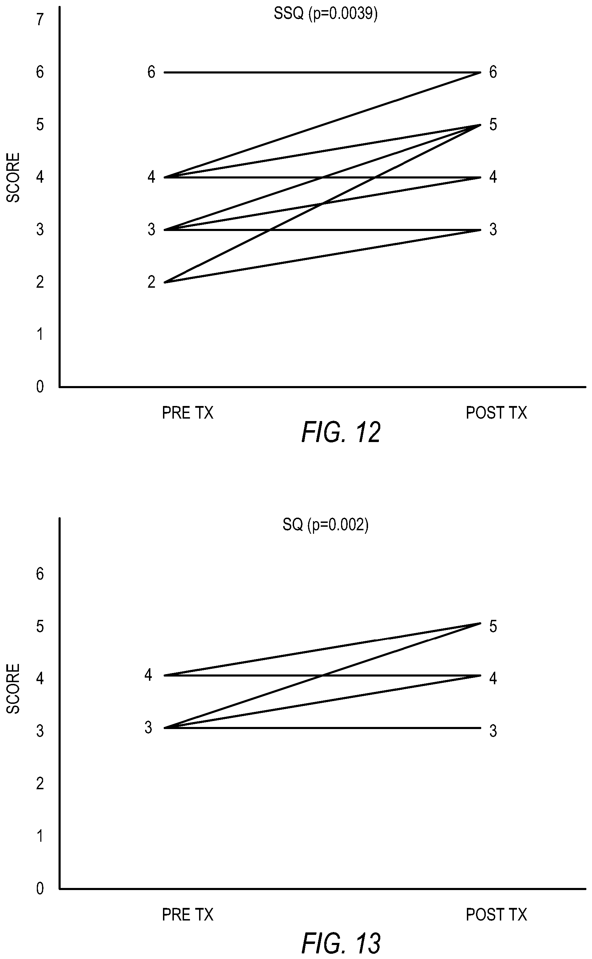

The method and device of the present invention could also be used to treat sexual satisfaction. **FIGs. 12 and 13** illustrates preliminary data for 14 patients from a clinical trial (NTC03280446 Clinicaltrials.gov) using techniques as described herein. The scores prior to treatment (PRE TX) and after treatment (POST TX) reveal a statically significant improvement on the Sexual Satisfaction Domain of the FSFI as well as the Sexual Satisfaction Questionnaire (SSQ).

### 11. Treating Painful Intercourse

The method and device of the present invention could also be used to treat painful intercourse (dyspareunia). Preliminary data for 22 patients from a clinical trial (NTC03280446 Clinicaltrials.gov) using techniques as described herein. The scores prior to treatment (PRE TX) and after treatment (POST TX) reveal a statically significant improvement on the Pain Domain of the FSFI.

It will be appreciated that the term "present invention" as used herein should not be construed to mean that only a single invention having a single essential element or group of elements is presented. Similarly, it will also be appreciated that the term "present invention" encompasses a number of separate innovations which can each be considered separate inventions. Although the present invention has thus been described in detail with regard to the preferred embodiments and drawings thereof, it should be apparent to those skilled in the art that various adaptations and modifications of the present invention may be accomplished without departing from the scope of the invention. Accordingly, it is to be understood that the detailed description and the accompanying drawings as set forth hereinabove are not intended to limit the breadth of the present invention, which should be inferred only from the following claims.

**Claims**

**1.**    A medical device (10) for at least one of treating female urinary incontinence, treating fecal incontinence, and vaginoplasty, comprising:

an elongated wand portion (12) having a generally cylindrical shape, the wand having a proximal end (32) and a distal end (30);

a fractional radiofrequency (RF) array proximate the distal end (30) of the wand (12), the fractional RF array having a plurality of electrodes (20) distributed over an area of greater than 1 cm$^2$ for delivering RF energy to a target tissue of a patient; and

dielectric material covering one or more electrodes (20) within the RF array such that said one or more electrodes (20) are electrically insulated from the target tissue of the patient, and energy is transmitted from the one or more electrodes (20) to said target tissue of the patient via capacitive coupling;

wherein the device (10) further comprises an impedance sensor for measuring an impedance from said electrodes (20) through said target tissue; and a feedback control loop for controlling how much RF energy is delivered through said electrodes (20) in order to maintain a temperature of said target tissue at a desired temperature, wherein said feedback control loop maintains a temperature of said target tissue of 40° to 44° C; and wherein the device further comprises a plurality of visible circumferential gradations (18) on said elongated wand portion (12), the circumferential gradations (18) being spaced a distance apart that approximately equals a width of said fractional RF array, such that when the fractional RF array is inserted inside a patient a doctor can rotate the wand (12) by one circumferential gradation (18) at a time for achieving substantial uniform RF energy exposure of the target tissue as the wand (12) is rotated without the doctor being able to see either the fractional RF array or the target tissue that is being exposed to the RF energy, optionally wherein said visible circumferential gradations (18) comprise grooves;

or wherein the device (10) further comprises a plurality of visible longitudinal gradations (16) on said elongated wand portion, the longitudinal gradations (16) being spaced a distance apart that approximately equals a longitudinal length of said fractional RF array, such that when the fractional RF array is inserted inside a patient a doctor can insert or retract the wand (12) one longitudinal gradation (16) at a time for achieving substantial uniform RF energy exposure of the target tissue without being able to see either the fractional RF array or the target tissue that is being exposed to the RF energy.

2. The medical device (10) of claim 1 wherein said fractional RF array is flat.

3. The medical device (10) of claim 1 wherein said fractional RF array is curved.

**Patentansprüche**

1. Medizinisches Gerät (10) für mindestens eines von Behandlung von Harninkontinenz bei Frauen, Behandlung von Stuhlinkontinenz und Vaginoplastik, umfassend:

einen länglichen Stababschnitt (12) mit einer allgemein zylindrischen Form, wobei der Stab ein proximales Ende (32) und ein distales Ende (30) aufweist;
ein fraktionales Radiofrequenz(RF)-Array nahe dem distalen Ende (30) des Stabs (12), wobei das fraktionale RF-Array eine Vielzahl von Elektroden (20) aufweist, die über eine Fläche von mehr als 1 cm$^2$ verteilt sind, um RF-Energie an ein Zielgewebe eines Patienten abzugeben; und

dielektrisches Material, das eine oder mehrere Elektroden (20) innerhalb des RF-Arrays abdeckt, sodass die eine oder mehrere Elektroden (20) elektrisch von dem Zielgewebe des Patienten isoliert sind und Energie aus der einen oder mehreren Elektroden (20) an das Zielgewebe des Patienten mittels kapazitiver Kopplung übertragen wird;
wobei das Gerät (10) ferner einen Impedanzsensor zum Messen einer Impedanz aus den Elektroden (20) durch das Zielgewebe umfasst; und eine Rückkopplungssteuerschleife zum Steuern, wie viel RF-Energie durch die Elektroden (20) abgegeben wird, um eine Temperatur des Zielgewebes auf einer gewünschten Temperatur zu halten, wobei die Rückkopplungssteuerschleife eine Temperatur des Zielgewebes von 40° bis 44° C aufrechterhält;
und wobei das Gerät ferner eine Vielzahl von sichtbaren umfänglichen Abstufungen (18) auf dem länglichen Stababschnitt (12) umfasst, wobei die umfänglichen Abstufungen (18) in einem Abstand beabstandet sind, der ungefähr gleich einer Breite des fraktionalen RF-Arrays ist, sodass, wenn das fraktionale RF-Array in einen Patienten eingeführt wird, ein Arzt den Stab (12) eine umfängliche Abstufung (18) auf einmal drehen kann, um eine im Wesentlichen gleichmäßige RF-Energieexposition des Zielgewebes zu erreichen, während der Stab (12) gedreht wird, ohne dass der Arzt entweder das fraktionale RF-Array oder das Zielgewebe, das der RF-Energie ausgesetzt wird, sehen kann, optional, wobei die sichtbaren umfänglichen Abstufungen (18) Rillen umfassen;
oder wobei das Gerät (10) ferner eine Vielzahl von sichtbaren Längsabstufungen (16) auf dem länglichen Stababschnitt umfasst, wobei die Längsabstufungen (16) in einem Abstand beabstandet sind, der ungefähr gleich einer Längslänge des fraktionalen RF-Arrays ist, sodass, wenn das fraktionale RF-Array in einen Patienten eingeführt wird, ein Arzt den Stab (12) eine Längsabstufung (16) auf einmal einführen oder zurückziehen kann, um eine im Wesentlichen gleichmäßige RF-Energieexposition des Zielgewebes zu erreichen, ohne entweder das fraktionale RF-Array oder das Zielgewebe, das der RF-Energie ausgesetzt wird, sehen zu können.

2. Medizinisches Gerät (10) nach Anspruch 1, wobei das fraktionale RF-Array flach ist.

3. Medizinisches Gerät (10) nach Anspruch 1, wobei das fraktionale RF-Array gekrümmt ist.

**Revendications**

1. Dispositif médical (10) destiné à au moins un traitement parmi le traitement de l'incontinence urinaire féminine, le traitement de l'incontinence fécale et la vaginoplastie, comprenant :

une partie de baguette allongée (12) présentant une forme généralement cylindrique, la baguette possédant une extrémité proximale (32) et une extrémité distale (30) ;
un réseau de radiofréquence (RF) fractionnaire à proximité de l'extrémité distale (30) de la baguette (12), le réseau RF fractionnaire comportant une pluralité d'électrodes (20) réparties sur une surface supérieure à 1 cm$^2$ pour délivrer de l'énergie RF à un tissu cible d'un patient ;
et
un matériau diélectrique recouvrant une ou plusieurs électrodes (20) au sein du réseau RF de sorte que lesdites une ou plusieurs électrodes (20) soient électriquement isolées du tissu cible du patient et l'énergie soit transmise desdites une ou plusieurs électrodes (20) audit tissu cible du patient par couplage capacitif ;
ledit dispositif (10) comprenant en outre un capteur d'impédance destiné à mesurer l'impédance desdites électrodes (20) à travers ledit tissu cible ; et une boucle de commande de rétroaction destinée à commander la quantité d'énergie RF délivrée à travers lesdites électrodes (20) afin de maintenir la température dudit tissu cible à une température souhaitée, ladite boucle de commande de rétroaction maintenant la température dudit tissu cible de 40° à 44°C ;
et ledit dispositif comprenant en outre une pluralité de gradations circonférentielles visibles (18) sur ladite partie de baguette allongée (12), lesdites gradations circonférentielles (18) étant espacées d'une distance qui est approximativement égale à la largeur dudit réseau RF fractionnaire, de sorte que, lorsque le réseau RF fractionnaire est inséré à l'intérieur d'un patient, un médecin puisse faire tourner la baguette (12) d'une gradation circonférentielle (18) à la fois pour obtenir une exposition à l'énergie RF uniforme substantielle du tissu cible lorsque la baguette (12) est tournée sans que le médecin puisse voir soit le réseau RF fractionnaire soit le tissu cible qui est exposé à l'énergie RF, éventuellement lesdites gradations circonférentielles visibles (18) comprenant des rainures ;
ou ledit dispositif (10) comprenant en outre une pluralité de gradations longitudinales visibles (16) sur ladite partie de baguette allongée, lesdites gradations longitudinales (16) étant espacées d'une distance qui est approximativement égale à la longueur longitudinale dudit réseau RF fractionnaire, de sorte que lorsque le réseau RF fractionnaire est inséré à l'intérieur d'un patient, un médecin puisse insérer ou rétracter la baguette (12) d'une gradation longitudinale (16) à la fois pour obtenir une exposition à l'énergie RF uniforme substantielle du tissu cible sans pouvoir voir soit le réseau RF fractionnaire soit le tissu cible qui est exposé à l'énergie RF.

2. Dispositif médical (10) selon la revendication 1, ledit réseau RF fractionnaire étant plat.

3. Dispositif médical (10) selon la revendication 1, ledit réseau RF fractionnaire étant incurvé.

*FIG. 1*

*FIG. 2*

FIG. 3

FIG. 4

FIG. 5

FIG. 6

FIG. 7

FIG. 8

FIG. 9

FIG. 10

FIG. 11

*FIG. 12*

*FIG. 13*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016263388 A1
- WO 2007092610 A2
- US 8540705 B
- US 8961511 B

**Non-patent literature cited in the description**

- **R. GUDIVAKA et al.** Effect of Skin Temperature on Multifrequency Bioelectrical Impedance Analysis. *Journal of Applied Physiology*, August 1996, vol. 81 (2), 838-45
- **WALL, MS et al.** *J Shoulder Elbow Surg.*, July 1999, vol. 8 (4), 339-44
- **PORTMAN**. *Maturutas*, 2014
- **OZKAN D et al.** *Anaesthesist*, 26 January 2016, vol. 65 (2), 134-6